# EUROPÄISCHE PATENTANMELDUNG

(11) **EP 2 813 263 A1**
(43) Veröffentlichungstag der Anmeldung: **17.12.2014**
(21) Anmeldenummer: 13002991.1
(22) Anmeldetag: 12.06.2013
(51) Int. Cl.: A61N 1/372, A61N 1/375

(54) **Herzschrittmachersystem mit einer Haltevorrichtung**

(71) Anmelder: Gutersohn, Achim, 49377 Vechta (DE)
(72) Erfinder: Gutersohn, Achim, 49377 Vechta (DE)
(74) Vertreter: Hauck Patent- und Rechtsanwälte

(57) **Zusammenfassung**

Herzschrittmachersystem mit
• einem Steuergerät, das eine Energiequelle, eine elektronische Steuerung und eine erste elektrische Kontaktfläche aufweist,
• einer ersten Sonde zur Anordnung in oder an einer ersten Herzkammer, gekennzeichnet durch
• eine in ein Blutgefäß einsetzbare Haltevorrichtung,
• ein Halteelement zur lösbaren Befestigung des Steuergeräts an der Haltevorrichtung und
• ein erstes elektrisches Kontaktelement, das elektrisch mit der ersten Sonde verbunden ist und das bei an der Haltevorrichtung befestigtem Steuergerät im elektrischen Kontakt mit der ersten Kontaktfläche steht.

## Beschreibung

Die Erfindung betrifft ein Herzschrittmachersystem mit einem Steuergerät, das eine Energiequelle, eine elektronische Steuerung und eine erste elektrische Kontaktfläche aufweist sowie eine erste Sonde zur Anordnung in oder an einer ersten Herzkammer.

Derartige Herzschrittmachersysteme sind seit Jahrzehnten bekannt. Das Steuergerät enthält einen Impulsgeber und stimuliert über die Sonde einen Herzmuskel mithilfe von elektrischen Impulsen, um diesen zu Kontraktionen anzuregen. Bei bekannten Herzschrittmachersystemen können die Sonden eine Doppelfunktion aufweisen. Einerseits dienen sie zum Abgeben der elektrischen Impulse, andererseits leiten sie dem Steuergerät elektrische Potentiale aus dem Herzen zu, um dessen Funktion überwachen und beispielsweise auf Unregelmäßigkeiten reagieren zu können. Ebenfalls bekannt sind Zwei- oder Dreikammerherzschrittmachersysteme, bei denen mehrere Sonden in unterschiedlichen Herzkammern angeordnet werden. Auf diese Weise kann beispielsweise mithilfe einer getriggerten Stimulation eine Leitungsstörung zwischen Vorhof und Kammer überbrückt und erfolgreich behandelt werden. Moderne Herzschrittmachersysteme werden zur Behandlung der unterschiedlichsten Störungen eingesetzt, insbesondere für die kardiale Resynchronisationstherapie (CRT, cardiac resynchronization therapy).

Bei den meisten im Einsatz befindlichen Herzschrittmachern wird das Steuergerät subkutan in das Fettgewebe implantiert, häufig oberhalb einer Brust unterhalb des Schlüsselbeins oder auch unterhalb eines großen Brustmuskels. Der oder die Sonden werden in der Regel transvenös zu der oder den betreffenden Herzkammern geführt, was die Verwendung relativ langer elektrischer Leitungen erfordert.

Ebenfalls bekannt, beispielsweise aus der Druckschrift EP 2 471 447 A1, sind sogenannte leitungslose Herzschrittmachersysteme (leadless pacemaker). Diese weisen ein miniaturisiertes Steuergerät auf, das unmittelbar in oder an der betreffenden Herzkammer angeordnet wird. Zur Befestigung weisen die aus der Druckschrift bekannten Systeme ein spiralförmiges Verankerungselement auf, das unmittelbar im Herzmuskel verankert werden kann. Derartige Systeme sind zunächst nur zur Behandlung einer einzigen Herzkammer geeignet. Sollen mehrere Herzkammern, beispielsweise rechter Vorhof und rechter Ventrikel, in die Therapie einbezogen werden, können mehrere derartige Schrittmachersysteme implantiert werden, die untereinander kommunizieren müssen. Eine hierfür grundsätzlich mögliche Funkverbindung ist wegen des relativ hohen Energieverbrauchs nachteilig. Die genannte Druckschrift schlägt daher eine Kommunikation zwischen mehreren derartigen Schrittmachersystemen durch Leitung elektrischer Signale durch das Gewebe vor. Dennoch stellt der Energieverbrauch derartiger Herzschrittmachersysteme ein Problem dar, da wegen der erforderlichen Miniaturisierung nur sehr kleine Batterien eingesetzt werden können. Wird ein Batteriewechsel erforderlich, erfordert dies einen aufwendigen medizinischen Eingriff zur Explantation des gesamten Systems.

Zum Implantieren und Explantieren bekannter leitungsloser Herzschrittmachersysteme werden spezielle Implantiersysteme eingesetzt, wie beispielsweise in der Druckschrift WO 2012/082735 A1 beschrieben. Wegen der Verankerung im Herzmuskel besteht allerdings auch bei Verwendung derartiger Systeme stets das Risiko einer Schädigung des Herzmuskels, sowohl bei der Implantation als auch bei der Explantation.

Davon ausgehend ist es die Aufgabe der Erfindung, ein Herzschrittmachersystem mit einem Steuergerät und einer Sonde zur Verfügung zu stellen, das insbesondere für einen Batteriewechsel einen einfacheren Austausch des Steuergeräts ermöglicht und dadurch auch über längere Zeiträume einen zuverlässigen Betrieb vereinfacht.

Diese Aufgabe wird gelöst durch das Herzschrittmachersystem mit den Merkmalen des Anspruchs 1. Vorteilhafte Ausgestaltungen sind in den Unteransprüchen angegeben. Das Herzschrittmachersystem hat
- ein Steuergerät, das eine Energiequelle, eine elektronische Steuerung und eine erste elektrische Kontaktfläche aufweist,
- eine erste Sonde zur Anordnung in oder an einer ersten Herzkammer,
- eine in ein Blutgefäß einsetzbare Haltevorrichtung, die
- ein Halteelement zur lösbaren Befestigung des Steuergeräts an der Haltevorrichtung und
- ein erstes elektrisches Kontaktelement aufweist, das elektrisch mit der ersten Sonde verbunden ist und das bei an der Haltevorrichtung befestigtem Steuergerät im elektrischen Kontakt mit der ersten Kontaktfläche steht.

Die Energiequelle des Steuergeräts kann insbesondere eine Batterie sein. Die elektronische Steuerung ist dazu eingerichtet, über die erste elektrische Kontaktfläche Impulse zur Stimulation eines Herzmuskels abzugeben. Diese werden über die erste elektrische Kontaktfläche und das erste elektrische Kontaktelement an die erste Sonde geleitet. Gegebenenfalls ist die elektronische Steuerung zusätzlich dazu ausgebildet, von der ersten Sonde abgeleitete Signale auszuwerten. Hierzu kann die erste Sonde eine oder mehrere elektrische Leitungen aufweisen. Darüber hinaus kann die elektronische Steuerung eine Kommunikationseinrichtung zum Austausch von Daten mit einem externen Gerät aufweisen.

Die erste Sonde ist zur Anordnung in oder an einer ersten Herzkammer vorgesehen. Sie kann hierzu insbesondere über eine Vene an einen Herzmuskel der ersten Herzkammer herangeführt sein. Grundsätzlich ist auch eine Anordnung der ersten Sonde innerhalb der ersten Herzkammer möglich. Auch in diesem Fall kann die erste Sonde an dem betreffenden Herzmuskel angeordnet sein. Die erste Herzkammer kann insbesondere der linke Ventrikel sein.

Das erfindungsgemäße Herzschrittmachersystem weist eine in ein Blutgefäß einsetzbare Haltevorrichtung für das Steuergerät auf. Die Haltevorrichtung ist dabei insbesondere hinsichtlich ihrer Abmessungen an die Abmessungen des Blutgefäßes, in das die Vorrichtung eingesetzt werden soll, angepasst. Sie wird in das betreffende Blutgefäß eingesetzt oder implantiert und kann über einen längeren Zeitraum darin verbleiben, insbesondere auch bei einem Austausch des Steuergeräts.

Die Haltevorrichtung weist ein Halteelement zur lösbaren Befestigung des Steuergeräts an der Haltevorrichtung auf. Hierzu kann das Steuergerät korrespondierende Halteabschnitte aufweisen, die mit dem Halteelement zusammenwirken. Insbesondere kann das Steuergerät in die Haltevorrichtung eingesetzt werden oder darin einrasten. In dem an der Haltevorrichtung befestigten Zustand befindet sich das Steuergerät dann in einer relativ zu der Haltevorrichtung fest vorgegebenen Position.

Die Haltevorrichtung weist ein erstes elektrisches Kontaktelement auf, das elektrisch mit der ersten Sonde verbunden ist. Somit ist die erste Sonde bzw. ein Teil davon in die Haltevorrichtung integriert. Bei an der Haltevorrichtung befestigtem Steuergerät steht das erste elektrische Kontaktelement im elektrischen Kontakt mit der ersten Kontaktfläche. Dadurch wird beim Befestigen des Steuergeräts an der Haltevorrichtung automatisch ein elektrischer Kontakt zwischen dem Steuergerät und der ersten Sonde hergestellt. Es ist daher nicht erforderlich, bei einem Austausch des Steuergeräts die Positionierung der ersten Sonde in oder an der ersten Herzkammer zu verändern oder anzutasten. Dennoch befindet sich das Steuergerät viel näher als ein herkömmlicher, subkutan implantierter Herzschrittmacher an der ersten Herzkammer. Die Verbindungsleitungen zwischen Sonde und Steuergerät können daher wesentlich kürzer ausgeführt werden, was zu einem problemlosen Betrieb des Herzschrittmachersystems auch über längere Zeiträume beiträgt.

Die Erfindung vereinfacht den zum Austausch des Steuergeräts erforderlichen medizinischen Eingriff auf zweierlei Weise. Zum einen befindet sich das Steuergerät gegebenenfalls in einem relativ großen Blutgefäß, sodass es viel einfacher zugänglich ist als ein unmittelbar in oder an einer Herzkammer angeordnetes, leitungsloses Herzschrittmachersystem. Zum anderen ermöglicht das Halteelement der Haltevorrichtung eine lösbare Befestigung des Steuergeräts, sodass zum Einsetzen und Entnehmen des Steuergeräts eine einfache und zuverlässige, gegebenenfalls für einen mehrfachen Wechsel ausgelegte Schnittstelle vorhanden ist. Insbesondere muss das Gewebe des Patienten in der Umgebung des Herzschrittmachersystems für einen Austausch des Steuergeräts nicht angetastet werden.

In einer Ausgestaltung hat das Herzschrittmachersystem eine zweite Sonde zur Anordnung in oder an einer zweiten Herzkammer, wobei das Steuergerät eine zweite elektrische Kontaktfläche aufweist und die Haltevorrichtung ein zweites elektrisches Kontaktelement aufweist, das elektrisch mit der zweiten Sonde verbunden ist und an das bei an der Haltevorrichtung befestigtem Steuergerät im elektrischen Kontakt mit der zweiten Kontaktfläche steht. Auf diese Weise kann das Steuergerät ebenso einfach wie vorstehend für die erste Sonde beschrieben beim Befestigen an der Haltevorrichtung automatisch mit der zweiten Sonde verbunden werden. Dies ermöglicht einen Zweikammerbetrieb des Herzschrittmachersystems, wobei es gegenüber herkömmlichen, subkutan implantierten Steuergeräten nicht zu einem erhöhten Energieverbrauch kommt, da eine Kommunikation unterschiedlicher Steuergeräte über eine Funkverbindung oder auf sonstige Art und Weise nicht erforderlich ist. Es versteht sich, dass das Herzschrittmachersystem wahlweise, insbesondere für einen Dreikammerbetrieb, mit einer entsprechend ausgebildeten dritten Sonde zur Anordnung in oder an einer dritten Herzkammer ausgerüstet sein kann. In diesem Fall kann das Steuergerät eine dritte elektrische Kontaktfläche aufweisen und die Haltevorrichtung ein drittes elektrisches Kontaktelement, das elektrisch mit der dritten Sonde verbunden ist und das bei an der Haltevorrichtung befestigtem Steuergerät im elektrischen Kontakt mit der dritten Kontaktfläche steht. Die zweite Herzkammer kann insbesondere das linke Atrium sein, die dritte Herzkammer insbesondere der rechte Ventrikel.

In einer Ausgestaltung ist die Haltevorrichtung zum Einsetzen in einen Koronarvenensinus ausgebildet. Der Koronarvenensinus ist wegen seines relativ großen Durchmessers und seiner Anordnung in unmittelbarer Nähe zu den zu behandelnden Herzkammern zum Einsetzen der erfindungsgemäßen Haltevorrichtung besonders geeignet. Ein weiterer Vorteil besteht darin, dass über die mit dem Koronarvenensinus verbundenen weiteren Blutgefäße Sonden zu mehreren zu behandelnden Herzkammern geführt werden können.

In einer Ausgestaltung weist die Haltevorrichtung einen röhrenförmigen Körper auf, der im in das Blutgefäß eingesetzten Zustand an einer Gefäßwand anliegt. Auf diese Weise kann die Haltevorrichtung dauerhaft am gewünschten Ort in dem Blutgefäß angeordnet werden.

In einer Ausgestaltung weist der röhrenförmige Körper ein Geflecht, Gewebe oder Gerüst aus Draht oder Blech auf. Insbesondere kann der röhrenförmige Körper ein Stent sein. Elastizität und Festigkeit des röhrenförmigen Körpers können so gewählt sein, dass ein problemloses Einsetzen der Haltevorrichtung in das Blutgefäß an der gewünschten Stelle möglich ist und der röhrenförmige Körper nach dem Einsetzen zuverlässig Halt an der Gefäßwand findet. Der Draht oder das Blech können grundsätzlich aus einem beliebigen biokompatiblen Metall oder auch aus einem Kunststoffmaterial bestehen. Besonders geeignet ist Nitinol. Die Drähte können an Kreuzungspunkten miteinander verbunden sein, beispielsweise durch Klammern oder durch Verschweißen. Weist der röhrenförmige Körper ein Blech auf, kann dieses mit einer Vielzahl von Öffnungen versehen sein und beispielsweise vor oder nach dem Formen in die röhrenförmige Gestalt lasergeschnitten werden.

In einer Ausgestaltung ist das Halteelement so angeordnet, dass das daran befestigte Steuergerät im Inneren des röhrenförmigen Körpers angeordnet ist, sodass es allseitig einen Abstand zu dem röhrenförmigen Körper aufweist. Es verbleibt also ein ringförmiger Freiraum um das Steuergerät herum, was einen ausreichenden Blutfluss durch das Blutgefäß ermöglicht und Strömungsturbulenzen im Bereich der Gefäßwand, die langfristig zu Problemen führen können, weitgehend vermeidet.

In einer Ausgestaltung weist die Haltevorrichtung ein erstes Ende, ein zweites Ende und einen mittleren Abschnitt auf, wobei das Halteelement an dem mittleren Abschnitt angeordnet ist und die erste Sonde eine erste elektrische Leitung aufweist, die am ersten Ende aus der Haltevorrichtung herausgeführt ist. Der mittlere Abschnitt ist zwischen dem ersten Ende und dem zweiten Ende angeordnet. Ein Teil der ersten elektrischen Leitung ist also in die Haltevorrichtung integriert. Dieser Teil kann insbesondere von einem gegebenenfalls isolierten Draht der Haltevorrichtung selbst gebildet sein oder an der Haltevorrichtung befestigt. In beiden Fällen wird eine Bewegung der ersten elektrischen Leitung im Bereich der Haltevorrichtung vermieden, was einem Verschleiß der ersten elektrischen Leitung in diesem sensiblen Bereich entgegenwirkt.

In einer Ausgestaltung weist die zweite Sonde eine zweite elektrische Leitung auf, die am zweiten Ende aus der Haltevorrichtung herausgeführt ist. Insbesondere kann das zweite Ende dem ersten Ende gegenüberliegen. Dadurch ergeben sich zunächst für die zweite elektrische Leitung die im Zusammenhang mit der ersten elektrischen Leitung vorstehend erläuterten Vorteile. Zum anderen wird die Positionierung der beiden Sonden dadurch vereinfacht, dass die außerhalb der Haltevorrichtung anzuordnenden elektrischen Leitungen bereits an einer optimalen Position aus der Haltevorrichtung herausgeführt sind. Vermieden wird, dass erste und zweite elektrische Leitung aneinander reiben oder sich auf sonstige Weise gegenseitig nachteilig beeinflussen.

In einer Ausgestaltung weist das Haltelement einen Ring und mindestens eine von dem Ring in dessen Mitte weisende Querstrebe auf, wobei das Steuergerät an der mindestens einen Querstrebe befestigt wird. Der Außendurchmesser des Rings kann insbesondere ungefähr dem Innendurchmesser des zum Einsetzen der Haltevorrichtung vorgesehenen Blutgefäßes entsprechen. Der Ring kann insbesondere in Umfangsrichtung des Blutgefäßes an der Haltevorrichtung angeordnet sein. Der Ring kann fest mit dem röhrenförmigen Körper verbunden sein oder nachträglich in diesen eingesetzt und daran befestigt werden. Der Ring kann aus einem biokompatiblen Material bestehen oder ein solches Material aufweisen, beispielsweise Titan, Nitinol oder ein geeignetes Kunststoffmaterial. Die Querstrebe kann einteilig mit dem Ring ausgebildet oder an dem Ring befestigt sein. Sie weist in die Mitte des Rings, erstreckt sich jedoch nicht unbedingt bis zur Mitte hin. Möglich ist allerdings auch eine sich über den gesamten Durchmesser des Rings erstreckende Querstrebe. Die Querstrebe bietet die Möglichkeit, das Steuergerät an einer optimalen Position innerhalb des Rings bzw. der Haltevorrichtung zu befestigen.

In einer Ausgestaltung sind zwei zur Mitte des Rings weisende Querstreben vorhanden, die in einem Abstand voneinander an einander gegenüberliegenden

Positionen enden und dort jeweils eines der Kontaktelemente aufweisen. In diesem Fall kann das Steuergerät zwischen den freien Enden der beiden Querstreben eingesetzt werden. Die Querstreben können dann gleichzeitig die mechanische Befestigung des Steuergeräts bewerkstelligen und den elektrischen Kontakt zu den beiden Sonden herstellen.

In einer Ausgestaltung weist das Steuergerät ein zylindrisches, kapselförmiges Gehäuse auf. Der Durchmesser des Gehäuses kann geringer sein als der Außendurchmesser der Haltevorrichtung, insbesondere kann er weniger als 60% davon oder sogar weniger als 50% oder weniger als 40% davon betragen. Das Steuergerät kann eine langgestreckte Form aufweisen. Die beiden Enden des Steuergeräts können halbkugelförmig abgerundet sein oder leicht spitz zulaufen. Insbesondere kann das gesamte Gehäuse eine hydrodynamische Stromlinienform aufweisen. Das Gehäuse kann aus einem biokompatiblen Material bestehen, beispielsweise wie im Zusammenhang mit dem Ring bereits erläutert.

In einer Ausgestaltung weist das Steuergerät eine Öffnung zum Hindurchführen eines Führungsdrahts auf. Mithilfe des Führungsdrahts kann das Steuergerät durch ein Blutgefäß bis an den Ort der Haltevorrichtung vorgeschoben werden. Die Öffnung kann hierzu insbesondere eine Durchgangsöffnung sein. Sie kann insbesondere in Längsrichtung des Gehäuses verlaufen. Sie kann entlang einer Mittellängsachse des Gehäuses angeordnet sein oder bevorzugt nahe einem äußeren Rand des Gehäuses.

In einer Ausgestaltung weist das Steuergerät Führungsmittel auf, die mit dem Halteelement zusammenwirken, um das Steuergerät beim Anordnen an der Haltevorrichtung in eine definierte Befestigungsposition relativ zu der Haltevorrichtung zu führen. Die Führungsmittel können beispielsweise Führungsflächen, Führungsrippen, Führungsnuten oder Führungsstifte sein. Insbesondere können die Führungsmittel eine Schrägfläche aufweisen, die beim Annähern des Steuergeräts an das Halteelement eine Rotation des Steuergeräts um seine Längsachse bewirken. Das Halteelement kann ebenfalls Führungsflächen aufweisen, die mit dem Führungsmittel des Steuergeräts zusammenwirken.

In einer Ausgestaltung liegen die Kontaktflächen an dem Steuergerät einander gegenüber. Dies ist einerseits für eine elektrische Isolation der Kontaktflächen voneinander vorteilhaft. Andererseits ermöglicht es eine besonders zuverlässige elektrische Kontaktierung über die beiden elektrischen Kontaktelemente, weil von diesen auf die Kontaktflächen ausgeübte Kräfte beide elektrischen Kontakte gleichermaßen begünstigen.

Die Erfindung wird nachfolgend anhand eines in Figuren dargestellten Ausführungsbeispiels näher erläutert. Es zeigen:
- Fig. 1: ein erfindungsgemäßes Herzschrittmachersystem in einer perspektivischen, teilweise schematisch vereinfachten Darstellung,
- Fig. 2: einen Teil der Haltevorrichtung und das Steuergerät aus Fig. 1 in einer weiteren perspektivischen Darstellung,
- Fig. 3: eine weitere perspektivische Ansicht der in Fig. 2 dargestellten Elemente des Herzschrittmachersystems.

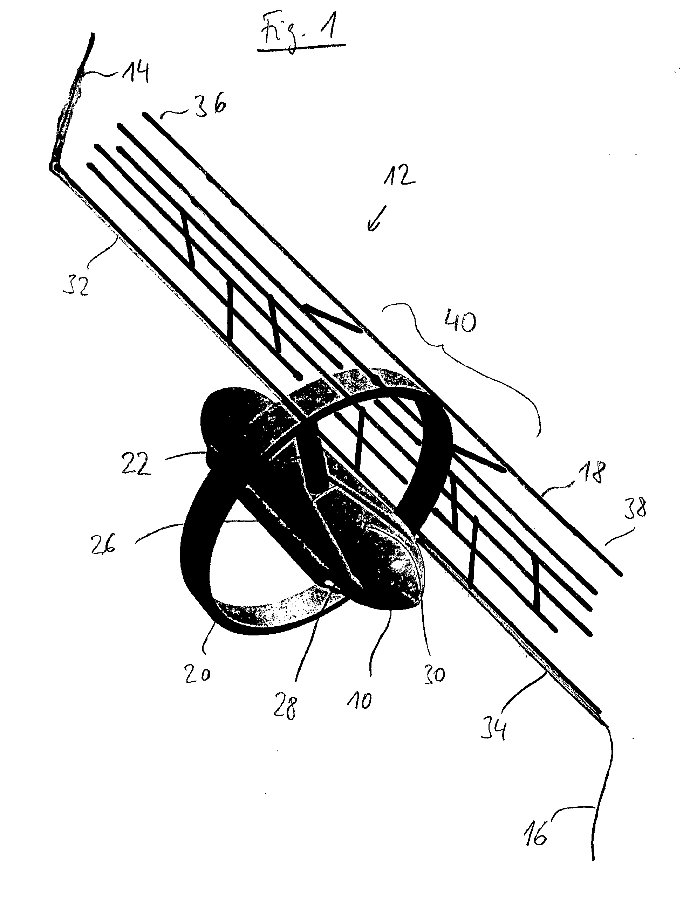

Das Herzschrittmachersystem aus Fig. 1 hat ein Steuergerät 10, eine Haltevorrichtung 12, eine erste Sonde 14 und eine zweite Sonde 16. Die Haltevorrichtung 12 weist einen käfigartigen, röhrenförmigen Körper 18 aus Nitinoldrähten auf. Dieser röhrenförmige Körper 18 ist in der Fig. 1 nur teilweise dargestellt. In Wirklichkeit ist der röhrenförmige Körper 18 im Querschnitt etwa kreisförmig und umgibt das Steuergerät 10 vollständig. Der röhrenförmige Körper 18 weist eine gewisse Elastizität auf und ist dazu ausgebildet, an der Innenseite einer Gefäßwand eines Blutgefäßes, in das die Haltevorrichtung eingesetzt wird, anzuliegen.

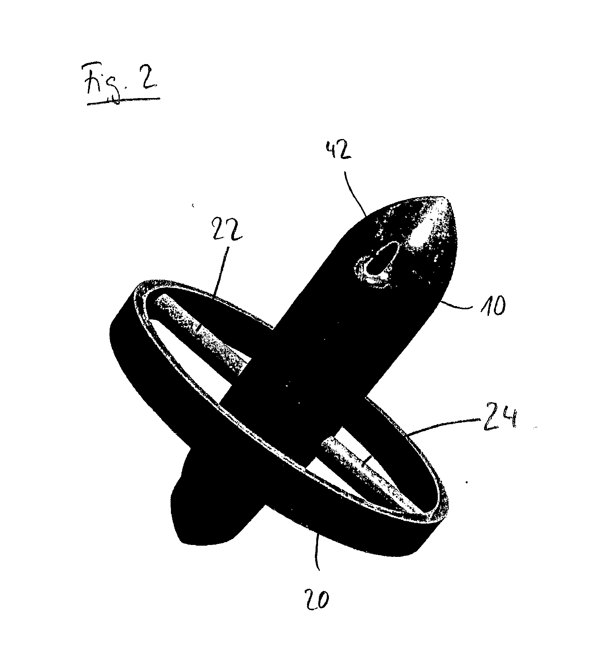

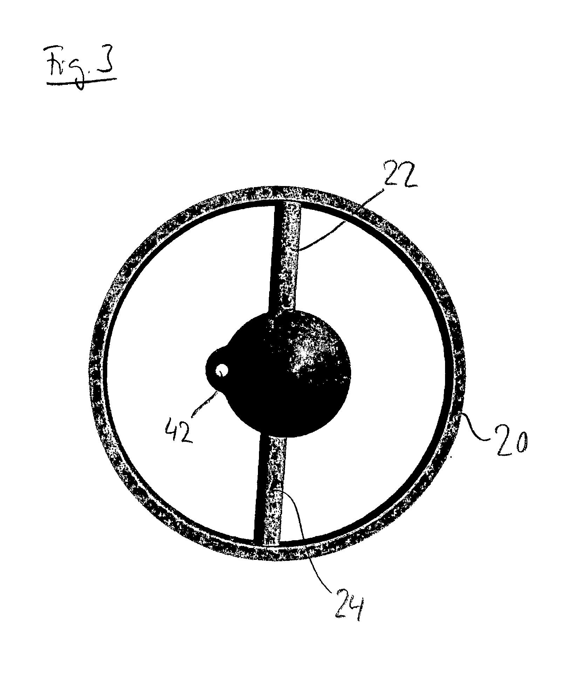

Die Haltevorrichtung 12 weist außerdem einen Ring 20 auf, der an der Innenseite des röhrenförmigen Körpers 18 befestigt ist. Ausgehend von der Innenseite des Rings 20 erstreckt sich eine erste Querstrebe 22 in Richtung zu der Mitte des Rings 20 hin. Diese endet in einem Abstand vom Mittelpunkt des Rings 20. Eine zweite Querstrebe 24 (siehe Fig. 2 und 3) ist in der Fig. 1 von dem Steuergerät 10 verdeckt.

Der Ring 20, die erste Querstrebe 22 und die zweite Querstrebe 24 bilden gemeinsam ein Halteelement für das Steuergerät 10, an dem das Steuergerät 10 lösbar befestigt ist. Hierzu weisen die beiden Querstreben 22, 24 jeweils an ihrem freien Ende einen Befestigungsabschnitt 26 auf. Die Befestigungsabschnitte 26 weisen zwei gegenüberliegende, unter einem Winkel zueinander angeordnete Schrägflächen auf, die beim Einsetzen des Steuergeräts 10 in die Haltevorrichtung 12 mit ebenfalls schräg angeordneten Führungsflächen 28 am Steuergerät 10 zusammenwirken. Jeweils zwei dieser Führungsflächen 28 sind auf jeder Seite des Steuergeräts 10 ausgebildet und begrenzen gemeinsam eine V-förmige Vertiefung 30 in der im Übrigen im Wesentlichen kreiszylindrischen Oberfläche des Gehäuses des Steuergeräts 10.

Teilweise in die Haltevorrichtung 12 integriert sind die erste Sonde 14 und die zweite Sonde 16. Die erste Sonde 14 weist eine erste elektrische Leitung 32 und die zweite Sonde 16 weist eine zweite elektrische Leitung 34 auf. Abschnitte dieser beiden Leitungen 32, 34 sind an einem Nitinoldraht des röhrenförmigen Körpers 18 entlanggeführt und daran befestigt. Sie weisen darüber hinaus jeweils einen Abschnitt auf, der über den röhrenförmigen Körper 18 übersteht. An den Enden dieser überstehenden Abschnitte werden die Sonden 14, 16 in elektrischen Kontakt mit den zu stimulierenden bzw. abzutastenden Herzmuskeln gebracht. Die Sonden 14, 16 sind in der Fig. 1 nur schematisch vereinfacht dargestellt.

Die erste elektrische Leitung 32 führt von einem ersten Ende 36 des röhrenförmigen Körpers 18 zu einem mittleren Abschnitt 40 davon und verläuft von dort weiter über den Ring 20 in die erste Querstrebe 22 hinein. Im Bereich des Befestigungsabschnitts 26 endet die erste elektrische Leitung 32 an einem nicht dargestellten ersten elektrischen Kontaktelement. Dieses steht bei an der Haltevorrichtung 12 befestigtem Steuergerät 10 im elektrischen Kontakt mit einer ebenfalls nicht im Einzelnen erkennbaren ersten Kontaktfläche des Steuergeräts 10. Entsprechend ist die zweite elektrische Leitung 34 von einem zweiten Ende 38, das dem ersten Ende 36 des röhrenförmigen Körpers 18 gegenüberliegt, hin zu dem mittleren Abschnitt 40 geführt und verläuft von dort über den Ring 20 und die zweite Querstrebe 24 bis hin zu einer zweiten Kontaktfläche, die an einem Befestigungsabschnitt 26 am freien Ende der zweiten Querstrebe 24 angeordnet ist. Sie gelangt bei in die Haltevorrichtung 12 eingesetztem Steuergerät 10 in elektrischen Kontakt mit einer am Steuergerät 10 ausgebildeten, zweiten Kontaktfläche.

Man erkennt in der Fig. 1, dass das Steuergerät 10 allseitig einen Abstand zu dem röhrenförmigen Körper 18 und dem Ring 20 aufweist. Durch den Freiraum zwischen dem Ring 20 und dem Steuergerät 10 ist eine ausreichende Blutzirkulation durch ein Blutgefäß, in das die Haltevorrichtung 12 eingesetzt ist, gewährleistet.

Weitere Einzelheiten des Steuergeräts 10 sind besser in den Fig. 2 und 3 erkennbar, in denen der röhrenförmige Körper 18 mit der ersten und zweiten Sonde 14, 16 nicht gezeigt ist. Man erkennt in der Fig. 2, dass das Steuergerät 10 ein zylindrisches, kapselförmiges Gehäuse mit einer langgestreckten Form aufweist. Das vordere und hintere Ende dieses Gehäuses sind leicht spitz auslaufend, stromlinienförmig abgerundet ausgebildet. An einer Seite des Gehäuses befindet sich eine Öffnung 42, die in Längsrichtung des Steuergeräts 10 durch dessen Gehäuse hindurch verläuft und durch die ein Führungsdraht hindurchgeführt werden kann. Die Öffnung 42 befindet sich in einem seitlichen Abstand von einer Mittellängsachse des Steuergeräts 10.

Ebenfalls gut in Fig. 2 zu erkennen ist die zweite Querstrebe 24 und dass das Steuergerät 10 zwischen der ersten Querstrebe 22 und der zweiten Querstrebe 24 gehalten ist.

In der Fig. 3 ist der Blick auf eines der Enden des Steuergeräts 10 gerichtet. Man erkennt ebenfalls die Halterung des Steuergeräts 10 zwischen der ersten Querstrebe 22 und der zweiten Querstrebe 24 und die durchgängige Öffnung 42 an einer Seite des Steuergeräts 10.

### Liste der verwendeten Bezugszeichen:

- 10: Steuergerät
- 12: Haltevorrichtung
- 14: Erste Sonde
- 16: Zweite Sonde
- 18: Röhrenförmiger Körper
- 20: Ring
- 22: Erste Quersterbe
- 24: Zweite Querstrebe
- 26: Befestigungsabschnitt
- 28: Führungsflächen
- 30: Vertiefung
- 32: Erste elektrische Leitung
- 34: Zweite elektrische Leitung
- 36: Erstes Ende
- 38: Zweites Ende
- 40: Mittlerer Abschnitt
- 42: Öffnung

## Patentansprüche

1. Herzschrittmachersystem mit
• einem Steuergerät (10), das eine Energiequelle, eine elektronische Steuerung und eine erste elektrische Kontaktfläche aufweist,
• einer ersten Sonde (14) zur Anordnung in oder an einer ersten Herzkammer, **gekennzeichnet durch**
• eine in ein Blutgefäß einsetzbare Haltevorrichtung (12),
• ein Halteelement zur lösbaren Befestigung des Steuergeräts (10) an der Haltevorrichtung (12) und
• ein erstes elektrisches Kontaktelement, das elektrisch mit der ersten Sonde (14) verbunden ist und das bei an der Haltevorrichtung (12) befestigtem Steuergerät (10) im elektrischen Kontakt mit der ersten Kontaktfläche steht.

2. Herzschrittmachersystem nach Anspruch 1, **gekennzeichnet durch** eine zweite Sonde (16) zur Anordnung in oder an einer zweiten Herzkammer, wobei das Steuergerät (10) eine zweite elektrische Kontaktfläche aufweist und die Haltevorrichtung (12) ei n zweites elektrisches Kontaktelement aufweist, das elektrisch mit der zweiten Sonde (16) verbunden ist und das bei an der Haltevorrichtung (12) befestigtem Steuergerät (10) im elektrischen Kontakt mit der zweiten Kontaktfläche steht.

3. Herzschrittmachersystem nach Anspruch 1 oder 2, **dadurch gekennzeichnet, dass** die Haltevorrichtung (12) zum Einsetzen in einen Koronarvenensinus ausgebildet ist.

4. Herzschrittmachersystem nach einem der Ansprüche 1 bis 3, **dadurch gekennzeichnet, dass** die Haltevorrichtung (12) einen röhrenförmigen Körper (18) aufweist, der im in das Blutgefäß eingesetzten Zustand an einer Gefäßwand anliegt.

5. Herzschrittmachersystem nach Anspruch 4, **dadurch gekennzeichnet, dass** der röhrenförmige Körper (18) ein Geflecht, Gewebe oder Gerüst aus Draht oder Blech aufweist.

6. Herzschrittmachersystem nach Anspruch 4 oder 5, **dadurch gekennzeichnet, dass** das Halteelement so angeordnet ist, dass das daran befestigte Steuergerät (10) im Inneren des röhrenförmigen Körpers (18) angeordnet ist, so dass es allseitig einen Abstand zu dem röhrenförmigen Körper (18) aufweist.

7. Herzschrittmachersystem nach einem der Ansprüche 1 bis 6, **dadurch gekennzeichnet, dass** die Haltevorrichtung (12) ein erstes Ende (36), ein zweites Ende (38) und einen mittleren Abschnitt (40) aufweist, wobei das Halteelement (12) an dem mittleren Abschnitt (40) angeordnet ist und die erste Sonde (14) eine erste elektrische Leitung (32) aufweist, die am ersten Ende (36) aus der Haltevorrichtung (12) herausgeführt ist.

8. Herzschrittmachersystem nach Anspruch 7, **dadurch gekennzeichnet, dass** die zweite Sonde (16) eine zweite elektrische Leitung (34) aufweist, die am zweiten Ende (38) aus der Haltevorrichtung (12) herausgeführt ist.

9. Herzschrittmachersystem nach einem der Ansprüche 1 bis 8, **dadurch gekennzeichnet, dass** das Haltelement einen Ring (20) und mindestens eine von dem Ring (20) in dessen Mitte weisende Querstrebe (22, 24) aufweist, wobei das Steuergerät (10) an der mindestens einen Querstrebe (22, 24) befestigt wird.

10. Herzschrittmachersystem nach Anspruch 9, **dadurch gekennzeichnet, dass** zwei zur Mitte des Rings (20) weisende Querstreben (22, 24) vorhanden sind, die in einem Abstand voneinander an einander gegenüberliegenden Positionen enden und dort jeweils eines der Kontaktelemente aufweisen.

11. Herzschrittmachersystem nach einem der Ansprüche 1 bis 10, **dadurch gekennzeichnet, dass** das Steuergerät (10) ein zylindrisches, kapselförmiges Gehäuse aufweist.

12. Herzschrittmachersystem nach einem der Ansprüche 1 bis 11, **dadurch gekennzeichnet, dass** das Steuergerät (10) eine Öffnung (42) zum Hindurchführen eines Führungsdrahts aufweist.

13. Herzschrittmachersystem nach einem der Ansprüche 1 bis 12, **dadurch gekennzeichnet, dass** das Steuergerät (10) Führungsmittel aufweist, die mit dem Halteelement zusammenwirken, um das Steuergerät (10) beim Anordnen an der Haltevorrichtung (12) in eine definierte Befestigungsposition relativ zu der Haltevorrichtung (12) zu führen.

14. Herzschrittmachersystem nach einem der Ansprüche 2 bis 13, **dadurch gekennzeichnet, dass** die Kontaktflächen an dem Steuergerät (10) einander gegenüberliegen.
